# EUROPEAN PATENT SPECIFICATION

(11) **EP 2 726 151 B1**
(45) Date of publication and mention of the grant of the patent: **05.08.2015**
(21) Application number: 12743199.7
(22) Date of filing: 28.06.2012
(51) Int. Cl.: A61N 7/00

(54) **TREATMENT APPARATUS FOR EXTERNAL APPLICATION TO A MAMMAL BODY**
BEHANDLUNGSVORRICHTUNG ZUR ÄUSSERLICHEN ANWENDUNG AN EINEM SÄUGETIERKÖRPER
APPAREIL DE TRAITEMENT POUR UNE APPLICATION EXTERNE À UN CORPS DE MAMMIFÈRE

(30) Priority: 29.06.2011 GB 201111012
(43) Date of publication of application: 07.05.2014
(73) Proprietor: Curar Animal Therapeutics Ltd, Newcastle upon Tyne, Tyne and Wear NE1 2DF (GB)
(72) Inventor: FLOYD, Helen, Ouseburn Newcastle upon Tyne NE1 2DF (GB); MASTERS, Peter, Ouseburn Newcastle upon Tyne NE1 2DF (GB)
(74) Representative: Olbrich, Thomas
(86) International application number: PCT/GB2012/051512
(87) International publication number: WO 2013/001302

(56) References cited:
- WO-A1-2004/037346
- WO-A2-2008/107842
- US-A1- 2005 065 531
- US-A1- 2009 287 085
- US-A1- 2010 168 620

## Description

The present invention relates to the field of non-invasive treatment apparatus. More specifically, the present invention relates to a device for the correct mounting and/or application of such treatment apparatus. Even more specifically, the present invention relates to a treatment apparatus for veterinary use.

### INTRODUCTION

Often when mammals or animals, sustain injuries, some kind of treatment or physical therapy is applied to improve the mammal's condition. Usually, a physician or veterinarian assesses the injury and decides which type and duration of treatment or therapy is best suited for respective mammal or animal to recover from the injury. Also, it is common practice today to apply more and more often therapy techniques and therapeutic devices suitable for humans to the injured mammal or animal, mainly because such therapies and devices have been clinically proven and are likely to be as effective to the mammal and animal physiology.

Animals and mammals do not tend to spend a long time in veterinary hospitals and are sent home with the owner rather earlier than later. Therefore, after the veterinarian's initial assessment, it is often up to the animal owner to carry out the treatment In addition, more human treatments are completed at home by unqualified people, due to the fact that many "safe" treatment devices are readily available for purchase.

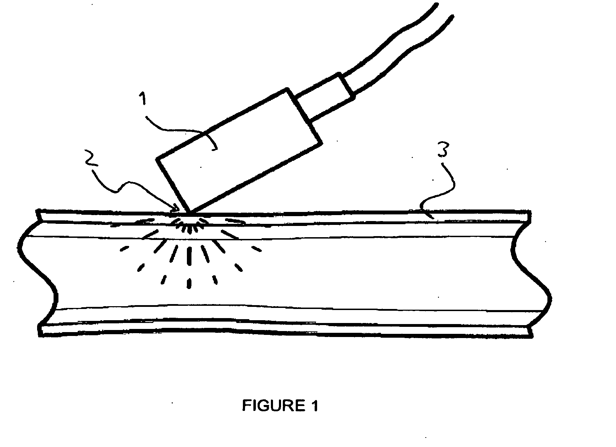

For example, after severe muscle or ligament injuries or fractures, the animal or mammal as well as the human may benefit from low intensity pulsed ultrasound treatment in order to accelerate the fracture repair process or accelerate the healing of tissue damage. Thus, the physician or veterinarian may decide to repeatedly apply an ultrasound probe to the injured area over a period of time. The probe may be manually held in place by the owner for the duration of the treatment or may be strapped to the injured area. Since it is unlikely for an animal or mammal to give any feedback and for unqualified people to provide meaningful feedback, the veterinarian, owner, physician or the self-applying patient may not be aware if the probe has been aligned correctly or if the probe has been strapped to the animal or mammal body too tightly, causing discomfort or even further injury to the animal. A misaligned probe 1 may also cause unwanted pressure concentration 2 on the animal or mammal body 3 as shown in Figure 1. Furthermore, a human patient applying an ultrasound probe to his injured area at home, may not be aware of correct alignment and pressure, potentially causing discomfort and/or reduced treatment effectiveness.

US 2005 0065531 A1 discloses a treatment apparatus with an spring-loaded transducer.

Also, medical or therapeutic devices that are used in human medicine and which are considered for veterinary use are often not suitable for use in the veterinary world. They are subject to different shear forces, different stresses or different types of interference (e.g. chewing or licking). In addition, these human devices may not be used in a clean, clinical environment and the injuries are often not clean or severely infected.

Typical devices that may be used for animal or mammal treatment include (i) LIPUS (Low Intensity Pulsed Ultrasound), (ii) wound management system, (iii) monitoring devices, (iv) diagnostic devices and (v) surgical tools.

Therefore, in order to allow such devices or apparatus to be applied to an animal or mammal, it is necessary to not only customise the devices for safe animal and mammal application, but also provide intelligent features that allow non-professionals to correctly mount and/or apply the device to an animal or mammal. When used on humans, this may also prevent people from applying a treatment device incorrectly potentially causing discomfort or even tissue damage.

Accordingly, it is an object of the present invention to provide a device that is capable of minimizing a misalignment, i.e. maximize the contact between the transducer and the body (i.e. animal, mammal and human body), and give intelligent feedback to the user during mounting or application of a treatment apparatus to a body.

### SUMMARY OF THE INVENTION

Preferred embodiments of the invention seek to overcome one or more of the above disadvantages of the prior art.

According to the present invention, there is provided a treatment apparatus for external application to a mammal body as defined in the appended claims.

This provides the advantage that the transducer element will self-align its output surface with the surface of the mammal body within a limited range, therefore maximising the transducer coupling, such as, for example the acoustic coupling between the transducer and the surface of the mammal body. Furthermore, the invention ensures that sufficient contact pressure is applied before the transducer element is activated for treatment, and potentially prevents applying a contact pressure that may harm the mammal by providing a feedback to the user if such harmful pressure is reached. Thus, the user will not apply too much force to the mammal body, either when strapping the treatment apparatus, such as an ultrasound probe, to the mammal body, or when manually pressing the treatment apparatus to the injured area. In particular, as soon as the contact force of the treatment apparatus exceeds a predetermined force, the feedback device triggers a signal indicating to the user that the applied pressure is too high, at which point the user can either release the applied manual pressure or loosen the strap, therefore preventing the mammal from discomfort or further injury. In addition, the present invention may prevent losing contact to the surface due to its ability to cope with contours and uneven pressure applied by unqualified / unskilled users.

The resilient coupling element is adapted to provide a limited range of three degrees of freedom between said transducer element and said housing.

Preferably, the first predetermined condition may be a contact pressure equal or above a first predetermined contact pressure applied by said transducer element to the mammal body in a normal direction with respect to the surface of the mammal body. The second predetermined condition may be a contact pressure below said first predetermined contact pressure applied by said transducer element to the mammal body in a normal direction with respect to the surface of the mammal body. The third predetermined condition may be a contact pressure above a second predetermined contact pressure applied by said transducer element to the mammal body in a normal direction with respect to the surface of the mammal body. Preferably, the first predetermined contact pressure may be smaller than said second predetermined contact pressure.

This provides the advantage of a sufficient and "safe" pressure range in which the apparatus operates; ensuring sufficient contact is made for maximum coupling, but alarming the user whenever the contact pressure exceeds a predetermined, potentially harmful contact pressure.

Advantageously, the predetermined condition may be defined by the resilience of said resilient coupling element.

The resilient coupling element is made of elastic material.

Preferably, the output may be an ultrasound signal.

The treatment apparatus may further comprise a data storage controller adapted to determine and log the number and duration of activation of said transducer element for a plurality of user profiles.

This provides the advantage that the user can access useful treatment information for different mammals. For example, a veterinarian may prescribe a 20 min treatment over a period of 6 weeks for a mammal. During treatment, the user may then be informed whether or not the treatment plan has been followed correctly. Also, at the end of the treatment, the veterinarian may access the logged treatment information to find out if his instructions were followed correctly.

The treatment apparatus may further comprise an external interface adapted to operatively connect to an external power source and/or external data storage device.

This provides the advantage that, whenever the device is stored, for example, in its storage container, it is re-charged and/or its logged information is transferred to a base unit. Preferably, the external interface may be a hot-shoe interface.

Additionally, the treatment apparatus may further comprise at least one proximity sensor adapted to determine the position of said treatment apparatus with respect to the mammal body, wherein said proximity sensor is adapted to selectively provide at least one second feedback signal in response to at least one predetermined position being measured by said at least one proximity sensor.

Preferably, the at least one first and second feedback signal may be any one or more of a visual and/or audible and/or tactile signal.

The treatment apparatus may further comprise a first transceiver adapted to transmit said first and/or second feedback signal to a remote second transceiver.

The said first transceiver may be further adapted to receive a control signal adapted to control the output of said transducer element.

The second feedback signal may be adapted to change in response to a change of position of the mammal treatment apparatus with respect to the mammal body during use.

Disclosed herein is also a feedback device for use with a mammal treatment apparatus, comprising at least one tactile sensor adapted to determine the force the mammal treatment apparatus applies to the mammal body when brought into contact with the mammal body, wherein said feedback device is further adapted to selectively provide at least one first feedback signal in response to at least one predetermined force being measured by said at least one tactile sensor.

This provides the advantage that the user will not apply too much force to the mammal body, either when strapping the treatment apparatus, such as an ultrasound probe, to the mammal body, or when manually pressing the treatment apparatus to the injured area. In particular, as soon as the contact force of the treatment apparatus exceeds a predetermined force, the feedback device triggers a signal indicating to the user that the applied pressure is too high, at which point the user can either release the applied manual pressure or loosen the strap, therefore preventing the mammal from discomfort or further injury.

The feedback device may further comprise at least one proximity sensor adapted to determine the position of the mammal treatment apparatus with respect to the mammal body, wherein said proximity sensor is adapted to selectively provide at least one second feedback signal in response to at least one predetermined position being measured by said at least one proximity sensor.

This provides the advantage that the treatment apparatus is always correctly positioned with respect to the mammal body. In particular, the mammal owner or unqualified user, who may not be familiar with the mammal physiology or the correct application of the device, may mount the treatment apparatus incorrectly by, for example, accidentally tilting the ultrasound transducer away from the target area. Also, the contact between the mammal body and the treatment apparatus may not be sufficient or misaligned. The proximity sensor of the feedback device is adapted to determine the exact position of the treatment apparatus with respect to the mammal body. In the event the treatment apparatus is not in a predetermined position, a signal is triggered indicating to the user that the apparatus needs repositioning.

The at least one first and second feedback signal may be one or more of a visual and/or audible and/or tactile signal.

This provides the advantage that the user can choose a preferred signal indication. For example, it may preferable to use a visual or tactile (vibration) indicator for very nervous animals, whereas an audible signal may be preferred if the user is unable to see the apparatus during application.

The feedback device may further comprise a first transceiver adapted to transmit said first and/or second feedback signal to a remote second transceiver. Preferably, the first transceiver may further adapted to receive a control signal adapted to control the output of the mammal treatment apparatus.

This provides the advantage that the correct application of the treatment apparatus can be monitored from a remote location. For example, the mammal may manipulate the position of the probe by licking, chewing or kicking, or the mammal my lie on the probe, therefore increasing the contact force of the probe to the mammal body. If that happens, the user can be notified by the signal even when not on site and prevent any serious damage to the mammal or the probe.

The first feedback signal may be adapted to change in response to a change of force the mammal treatment apparatus applies to the mammal body during use. The second feedback signal may be adapted to change in response to a change of position of the mammal treatment apparatus with respect to the mammal body during use.

This provides the advantage that the user will not only be notified by a characteristic signal in the event of exceeding a pressure threshold or when the probe is misaligned, but also when the pressure is below the threshold or the probe is in the correct position. Also, the change of the signal may also indicate to the user the extent of misalignment or exceeding pressure. For example, a low tone may indicate correct positioning or alignment, whereas a higher pitched tone may be used with increasing pressure or further misalignment This feature can help the user to home-in to the correct pressure or position of the treatment apparatus.

According to a further of the present invention, there is provided a treatment apparatus for external application to a mammal body, comprising:
at least one transducer element adapted to provide an output towards and/or into the mammal body, and
a feedback device according to the second aspect of the present invention.

Preferably, the output may be any one of an ultrasound signal, a vacuum, a water jet and an electromagnetic radiation. Furthermore, the feedback device may be removably coupled to said treatment apparatus, allowing the feedback device to be used with more than one treatment apparatus.

Alternatively, the feedback device may be an integral part of said treatment apparatus. Including the feedback device integrally with the treatment apparatus may allow a more compact and robust design.

### BRIEF DESCRIPTION OF THE DRAWINGS

A preferred embodiment of the present invention will now be described, by way of example only and not in any limitative sense, with reference to the accompanying drawings, in which:
**Figure 1** shows a simplified schematic side view of a known probe wrongly applied to a surface,
**Figure 2** shows a simplified schematic sectional side view of an example of the preferred embodiment of a treatment apparatus of the present invention,
**Figure 3** shows an alternative schematic sectional side view of the preferred embodiment of Figure 2 having a hand-held housing,
**Figure 4** shows an example of a base unit for storing the preferred embodiment of the hand-held treatment apparatus,
**Figure 5** shows the base unit and treatment apparatus of Figure 4, wherein the base unit includes a display for displaying information on the data stored in the treatment apparatus,
**Figure 6** shows the base unit of Figure 4 and an alternative embodiment of the treatment apparatus of the present invention, which is configured as an external adapted unit to be connected to a base system,
**Figure 7** shows a perspective view of an example of a second embodiment of the present invention as integral part of an ultrasound probe,
**Figure 8** shows a bottom view of the embodiment of Figure 7,
**Figure 9** shows a sectional side view along A-A in Figure 8, and
**Figure 10** shows a sectional side view along D-D in Figure 8.

### DETAILED DESCRIPTION OF EMBODIMENTS

It is understood that the terms "apparatus", "device", treatment apparatus" and "treatment device" have the same meaning and can be used interchangeably.

In addition, the following example describes the invention when used with animals. However, it is understood that different embodiments of the present invention are suitable for use with animal, mammals and humans. Preferably, the invention is for use on all mammals, including humans, however, it has significant benefits when used on non-human mammals.

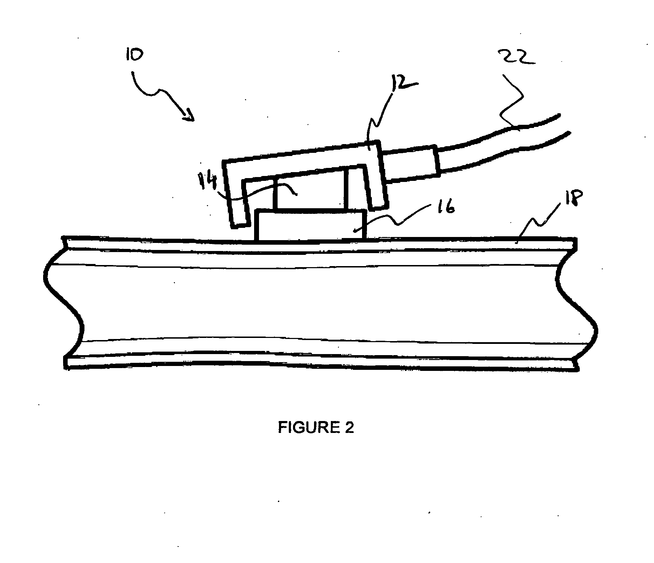

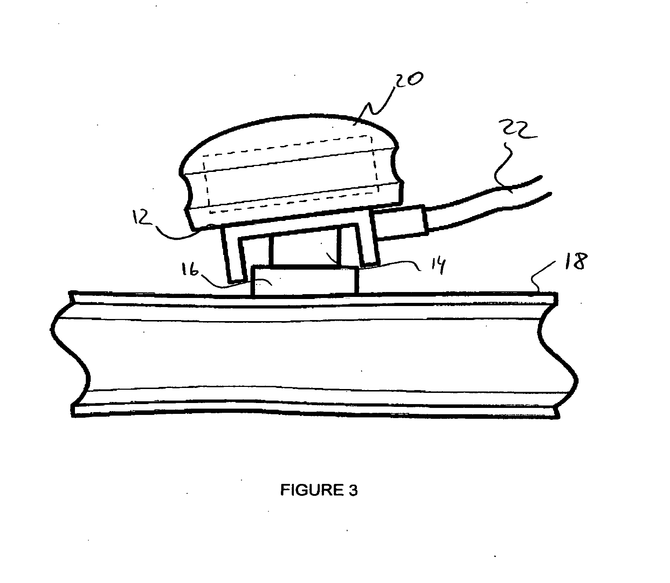

Referring now to Figures 2 and 3, a preferred embodiment of the present invention is disclosed in form of a hand-held ultrasound treatment apparatus 10. The treatment apparatus 10 consists of a housing 12, a resilient coupling element 14 and an ultrasound transducer 16. The housing 12 is shaped so as to accommodate the coupling element 14 and at least part of the transducer 16. A switch (not shown) operatively connects the transducer 16 to a power source (not shown), such as a battery (not shown). The switch (not shown) may be integrated within the coupling element 14 such that the switch (not shown) is operated by compressing the coupling element 14. For example, in order to deform the coupling element 14 and operated the switch (not shown), a predetermined contact pressure, defined by the resilience properties of the coupling element 14, has to be exerted by the user onto the surface 18 of the animal. At the same time, any misalignment of the treatment apparatus 10 with respect to the surface 18 of the animal is compensated by the deformable resilient coupling element 14, ensuring maximum acoustic coupling between the ultrasound transducer 16 and the surface 18 of the animal, as well as preventing unwanted pressure concentration and potential harm to the animal.

The coupling element 14 is preferably made of a flexible rubber, foam, thermoplastic elastomer or one or more springs. However, it is understood by the skilled person in the art that any suitable coupling element 14 adapted to deform at a predetermined pressure may be used. Also, it is further understood by the skilled person in the art that any suitable arrangement of the coupling element 14, the transducer 16 and switch (not shown) that enables the operation of the switch (not shown) at a predetermined contact pressure may be used.

Advantageously, the coupling element 14 couples the transducer 16 to an interior surface of the housing 12 such that the transducer 16 can move with respect to the housing 12 within a limited range of at least one degree of freedom. Preferably, the transducer 16 is coupled to the interior surface of the housing 12 such that the transducer 16 can move with respect to the housing 12 within a limited range of at least two degrees of freedom. Even more preferably, the transducer 16 is coupled to the interior surface of the housing 12 such that the transducer 16 can move with respect to the housing 12 within a limited range of three degrees of freedom.

Furthermore, the switch (not shown) is further adapted to trigger a feedback signal if the contact pressure between the treatment apparatus 10 and the surface 18 of the animal exceeds a predetermined pressure threshold. Preferably, the feedback signal activates an LED visually located on the external surface of the housing 12, but any other form of feedback may be used, such as, for example, and audio signal or a tactile signal (i.e. vibration). In particular, if the contact pressure between the treatment apparatus 10 and the surface 18 of the animal exceeds a predetermined threshold, the increased deformation of the resilient coupling element 14 will cause the switch (not shown) to activate the LED indicating a pressure that may be harmful to the animal. Alternatively, a second switch (not shown) may be used to be activated by the increased deformation of the resilient coupling element 14. Optionally, the feedback signal may be utilised to deactivate the transducer 16.

The housing 12 may be shaped so as to be held by one hand including a hand-held portion 20.

The treatment apparatus 10 may be powered by a power cable 22 connecting the hand-held treatment apparatus 10 to a base system (not shown) or external power source. Preferably, the treatment apparatus 10 is a stand-alone unit powered by a battery. Preferred output power of the transducer 16 may be about 100 mW / cm², but any other required power output may be used.

Optionally, the treatment apparatus 10 comprises data storage (not shown) and a controller (not shown) configured to determine duration and number of treatments and log the data for various different user profiles. For example, the operator may be able to set up user profiles for a number of animals. Each time animal 'A' is treated, the number and duration of that treatment is logged under the animal 'A' user profile. The stored data may be used to show to the operator whether or not the prescribed treatment has been followed.

Preferably, the treatment apparatus 10 is adapted to directly process the stored data and assess the data with regards to the prescribed treatment plan, providing, for example a colour-coded visual indicator showing a level of compliance to the prescribed treatment plan.

Alternatively, the stored data may be transferred to an external location for further analysis.

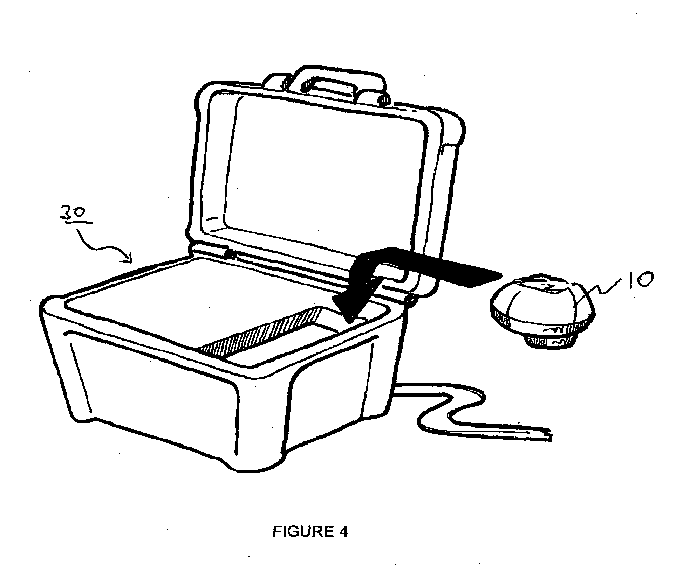

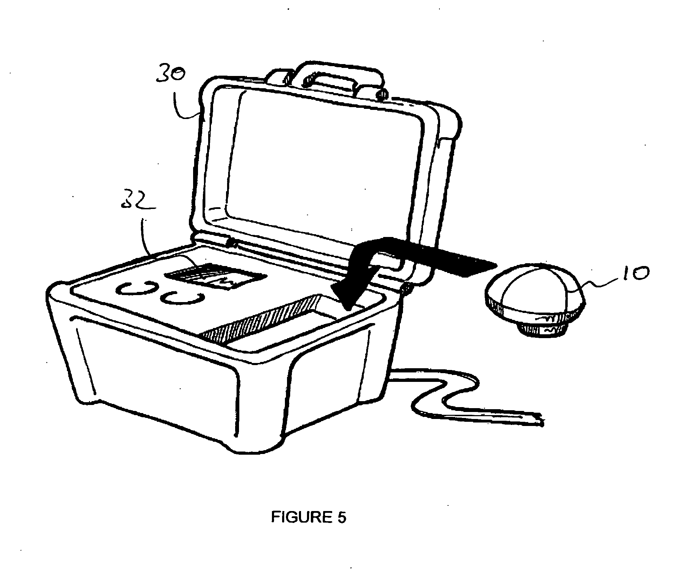

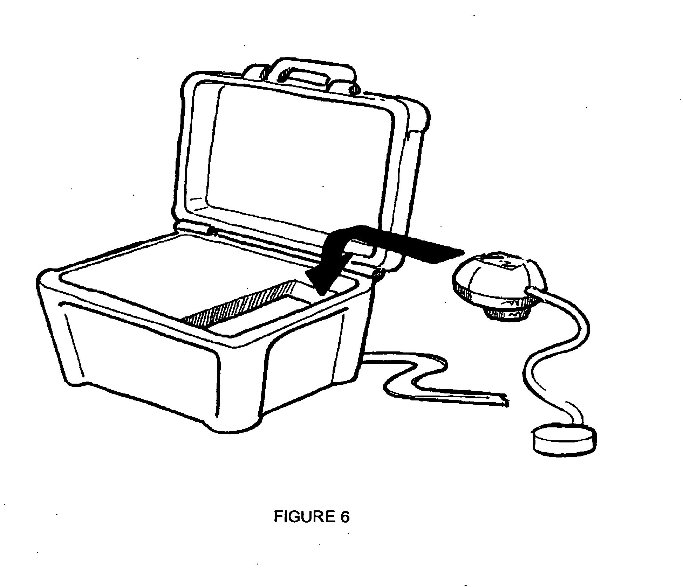

Figures 4 to 6 shows a base unit 30 for storing the hand-held treatment apparatus 10. The base unit 30 may simply perform the function of protecting the stand-alone hand-held treatment apparatus 10 during transport.

Preferably, the base unit 30 is adapted to re-charge the battery powered hand-held treatment apparatus 10. In particular, the hand-held treatment apparatus 10 may comprise external interface contacts (not shown) that operatively mate with a connector (e.g. hot-shoe connection, not shown) of the base unit 30. When the hand-held treatment apparatus 10 is stored and connected to the base unit 30, the battery of the hand-held treatment apparatus 10 can be charged through the interface contacts. Optionally, the interface contact are adapted to coexitingly transfer the stored data from the hand-held treatment apparatus 10 to the base unit 30, where a computer (not shown) may analyse the data and provide the results of the analysis on a display 32.

During use, the operator creates a user profile for the animal to be treated including a treatment plan (e.g. 20 min daily application for 6 weeks). The animal is then treated under its user profile. The operator then simply presses the transducer 16 contact surface of the treatment apparatus 10 against the surface 18 of the animal at the treatment area. An aqueous gel or any other acoustic coupling agent may be applied to the contact surface of the ultrasound probe. As soon as the contact pressure is equal or exceeds a first contact pressure threshold, the transducer 16 is activated providing, for example, an ultrasound output to the animal. In the event the operator misaligns the treatment apparatus 10 with respect to the surface 18 of the animal, the coupling element 14 compensates for the misalignment ensuring maximum contact area and acoustic coupling between the transducer 16 and the surface 18 of the animal. In addition, in the event the operator, or a strap, applies a potentially harmful pressure to the animal, an activated LED will indicate that to the operator, so that it can be corrected. The duration and number of treatment are logged by the treatment apparatus, which may also provides an instant indication, whether or not, any treatments have been missed and the duration of each treatment complies with the prescribed treatment plan. After use, the treatment apparatus 10 is secured by a hot-shoe connector in a base unit 30, where the treatment apparatus is re-charged and the data may be transferred to an onboard computer of the base unit 30.

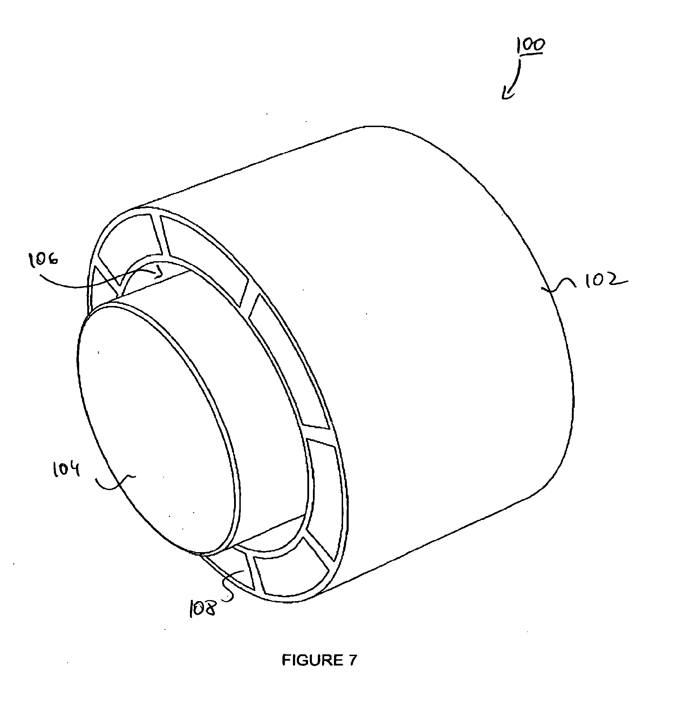

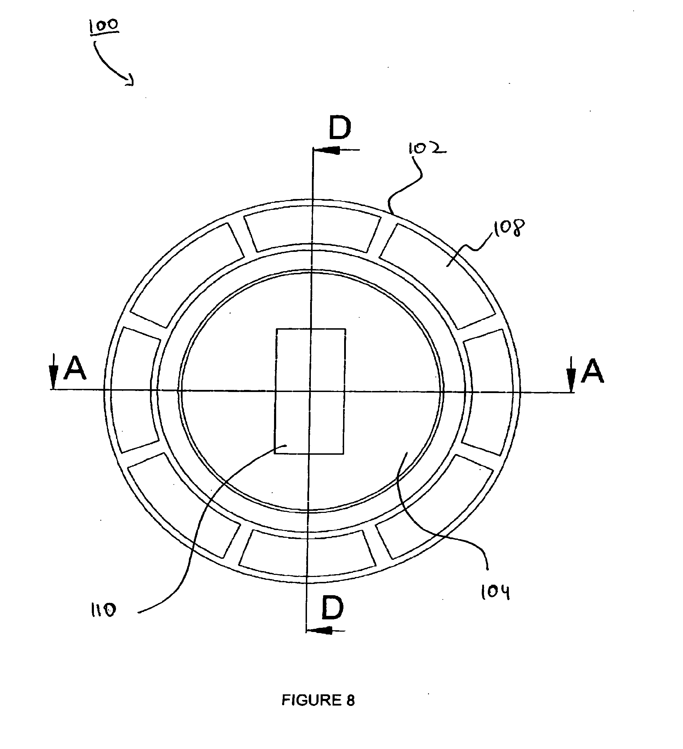

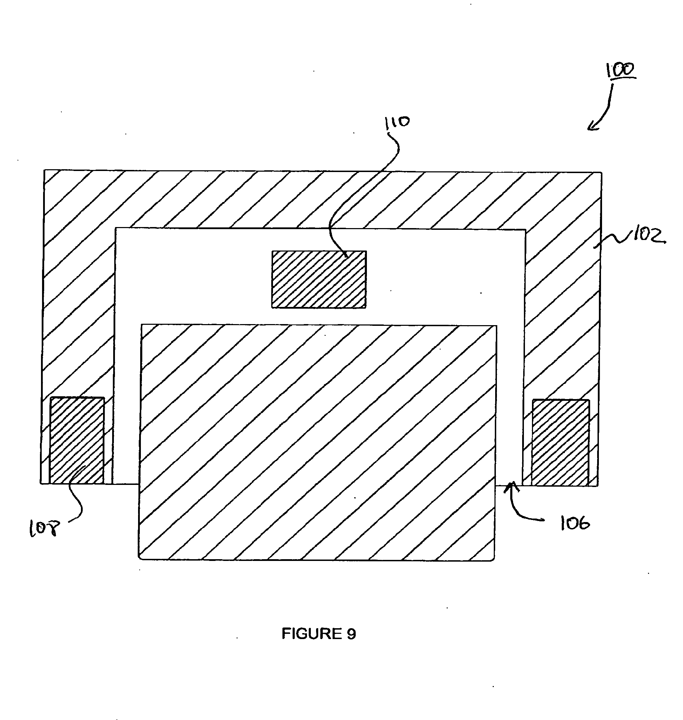

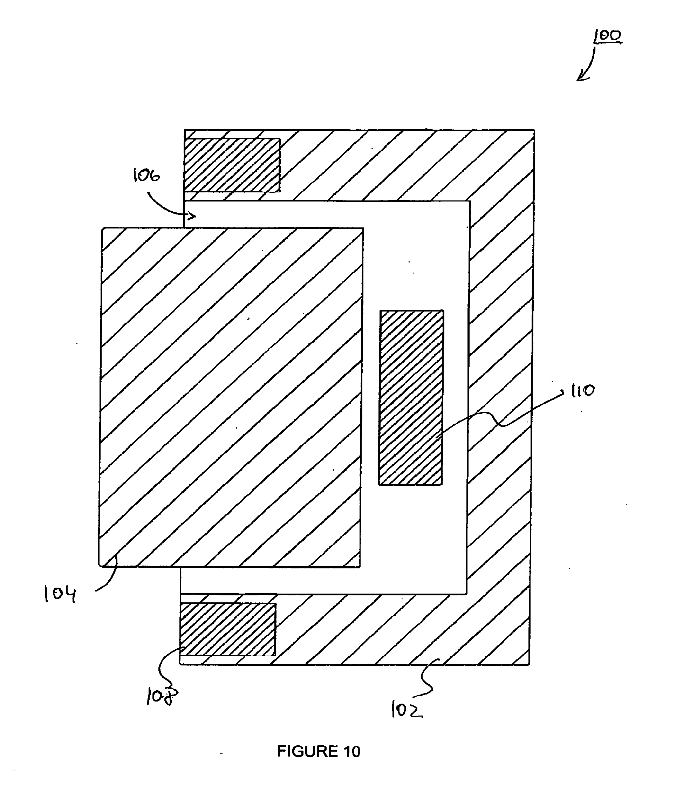

Referring to Figures 7 to 10, an alternative second embodiment of the present invention is disclosed where a feedback device is provided as integral part in an ultrasound probe 100. The probe 100 consists of a cylindrically shaped transducer housing 102, a tactile sensor 104 operatively coupled along a longitudinal axis inside the housing 102 such that at least part of the tactile sensor 104 project out of an opening 106 of the housing 102. The probe 100 further comprises an array of proximity sensors 108 arranged along the rim of the housing, wherein the proximity sensors are embedded inside the cylindrical side wall of the housing 102. An ultrasound transducer 110 is located inside the housing 102 and directed towards the opening 106.

The tactile sensor 104, the array of proximity sensors 108 and the ultrasound transducer 110 are all directed into the same direction, towards the opening of the housing 102.

During operation, the probe 100 is placed onto the injured area of the animal body, such that the output of the ultrasound transducer 110 is directed towards the animal body. Tactile sensor 104 and proximity sensors 108 are also directed towards the animal body. The probe is then pressed onto the injured area, wherein the tactile sensor 104 triggers a signal output in the event the pressure provided by the probe exceeds a predetermined pressure. The triggered signal output may be an audible noise, a visual indication such as a light, or vibration of the probe 100.

At the same time, the proximity sensors 108 measure the distance between the housing rim and the surface of the animal body. In the event the distance of one or more proximity sensors differs from the distance of other proximity sensors of the array, a signal output is triggered, indicating that the probe is not properly aligned with the surface of the injured area of the animal body. This allows the user to correct the position of the probe 100 until the signal is switched off. Similarly to the tactile sensor, the signal output may be a noise, a visual indication such as a light, or vibration of the probe 100.

Preferably, the signal of the tactile sensor 104 differs from the signal of the proximity sensor 108 such that the user is able to distinguish between them.

Alternatively, the probe may provide a continuous signal, e.g. a noise, which changes its characteristics according to the applied pressure or position. For example, at low contact pressure the probe 100 may provide a low frequency noise, which increases in frequency with increasing pressure. Similarly, a low frequency noise may indicate a correct position/alignment of the probe 100, wherein any misalignment of the probe increases the noise frequency.

The same effect can be achieved by a light source that changes colour in accordance to the pressure applied by the probe or the current alignment of the probe with respect to the animal body.

Preferably, the tactile sensor 104 is a load cell. The load cell may comprise a pressure sensor which may be used alone or in combination with other sensors to provide feedback to during application. By using these mechanisms together (or individually), it would allow the owner to position the probe precisely and safely. It would let you know when you are in the correct proximity of the animal and would then guide the owner as to how much pressure to apply to the probe. Pressure ranges may be used to define the applied pressure as *'good', 'poor',* or *'too much'.*

The proximity sensor 108 may use a capacitance or resistance sensing mechanism in order to provide feedback of how much of the probe rim is in contact with the animal. Ranges may be used to indicate if the contact of the probe is 'excellent' (e.g. 80%-100% contact), *good'* (70%-80% contact), *'sufficient'* (50%-70% contact) or *'insufficient'*(< 50% contact).

Alternatively, an array of infrared sensors may be used as proximity sensors 108 to detect the position of the probe 100 with respect to the animal body.

In yet another alternative embodiment, thermistors may be used to detect a heat signature from the animal. If one or more of the thermistors move away from the animal skin, the temperature would drop triggering a signal that the probe was out of contact

All these methods provide a read out to notify user of any deviation from the norm or if device is being used abnormally.

All of those feedback signals, visual, audible or tactile, provide a feedback to the user of any deviation form a predetermined norm in order to ensure that the treatment is delivered effectively and without harming the animal. Such a feedback from a pressure sensor alone or in combination with a proximity sensor, allows the user to position the probe precisely and safely.

In yet another alternative embodiment of the present invention, the feedback device (not shown), probe 100 or treatment apparatus may comprise a transceiver (not shown) that allows the feedback signals to be transmitted to a remote location so that the user can monitor the treatment without having to be onsite. The transceiver may also be able to receive a control signal for the transducer allowing the user to control the output of the transducer from a remote location.

It is understood that any suitable probe or treatment apparatus may be used in combination with the present invention. Furthermore, the present invention may be in the form of a feedback device that can be removably coupled to an existing treatment apparatus, or it may be an integral part of a probe or treatment apparatus.

It will be appreciated by persons skilled in the art that the above embodiments has been described by way of example only and not in any limitative sense, and that various alterations and modifications are possible without departing from the scope of the invention as defined by the appended claims. In particular, the treatment apparatus may include other treatment means adapted to provide other types of treatment such as radiation treatment, water jet treatment or vacuum treatment.

## Claims

1. A treatment apparatus for external application to a mammal body, comprising:
a housing,
a transducer element, adapted to provide an output towards and/or into the mammal body,
a switch element, adapted to activate said transducer element in accordance with a first predetermined condition and deactivate said transducer element in accordance with a second predetermined condition,
a feedback element, adapted to provide at least one first feedback signal in response to at least a third predetermined condition, and
a resilient coupling element, made of elastic material and adapted to operatively couple said transducer element to said housing and provide a limited range of three degrees of freedom between said transducer element and said housing.

2. A treatment apparatus according to claim 1, wherein said first predetermined condition is a contact pressure equal or above a first predetermined contact pressure applied by said transducer element to the mammal body in a normal direction with respect to the surface of the mammal body.

3. A treatment apparatus according to claim 2, wherein said second predetermined condition is a contact pressure below said first predetermined contact pressure applied by said transducer element to the mammal body in a normal direction with respect to the surface of the mammal body.

4. A treatment apparatus according to any one of the preceding claims, wherein said third predetermined condition is a contact pressure above a second predetermined contact pressure applied by said transducer element to the mammal body in a normal direction with respect to the surface of the mammal body.

5. A treatment apparatus according to claims 2 to 4, wherein said first predetermined contact pressure is smaller than said second predetermined contact pressure.

6. A treatment apparatus according to any one of the preceding claims, wherein said predetermined condition is defined by the resilience of said resilient coupling element.

7. A treatment apparatus according to any one of the preceding claims, wherein said output is an ultrasound signal.

8. A treatment apparatus according to any one of the preceding claims, further comprising a data storage controller adapted to determine and log the number and duration of activation of said transducer element for a plurality of user profiles.

9. A treatment apparatus according to any one of the preceding claims, further comprising an external interface adapted to operatively connect to an external power source and/or external data storage device.

10. A treatment apparatus according to claim 9, wherein said external interface is a hot-shoe interface.

11. A treatment apparatus according to any one of the preceding claims, further comprising at least one proximity sensor adapted to determine the position of said treatment apparatus with respect to the mammal body, wherein said proximity sensor is adapted to selectively provide at least one second feedback signal in response to at least one predetermined position being measured by said at least one proximity sensor.

12. A treatment apparatus according to claim 11, wherein said at least one first and second feedback signal is any one or more of a visual and/or audible and/or tactile signal.

13. A treatment apparatus according to any one of claims 11 and 12, further comprising a first transceiver adapted to transmit said first and/or second feedback signal to a remote second transceiver.

14. A treatment apparatus according to claim 13, wherein said first transceiver is further adapted to receive a control signal adapted to control the output of said transducer element.

15. A treatment apparatus according to any one of claims 11 to 14,
wherein said second feedback signal is adapted to change in response to a change of position of the mammal treatment apparatus with respect to the mammal body during use.

## Patentansprüche

1. Eine Behandlungsvorrichtung zur externen Anwendung an einem Säugetierkörper, beinhaltend:
ein Gehäuse,
ein Wandlerelement, das angepasst ist, um eine Ausgabe in Richtung des Säugetierkörpers und/oder in diesen bereitzustellen,
ein Schalterelement, das angepasst ist, um das Wandlerelement gemäß einem ersten vorbestimmten Zustand zu aktivieren und das Wandlerelement gemäß einem zweiten vorbestimmten Zustand zu deaktivieren,
ein Rückmeldeelement, das angepasst ist, um als Reaktion auf mindestens einen dritten vorbestimmten Zustand mindestens ein erstes Rückmeldesignal bereitzustellen, und
ein nachgiebiges Kopplungselement, das aus elastischem Material hergestellt und angepasst ist, um das Wandlerelement betriebsfähig an das Gehäuse zu koppeln und zwischen dem Wandlerelement und dem Gehäuse einen eingeschränkten Bereich von drei Freiheitsgraden bereitzustellen.

2. Behandlungsvorrichtung gemäß Anspruch 1, wobei der erste vorbestimmte Zustand ein Kontaktdruck ist, der gleich oder höher als ein erster vorbestimmter Kontaktdruck ist, der durch das Wandlerelement in einer senkrechten Richtung in Bezug auf die Oberfläche des Säugetierkörpers auf den Säugetierkörper angewendet wird.

3. Behandlungsvorrichtung gemäß Anspruch 2, wobei der zweite vorbestimmte Zustand ein Kontaktdruck ist, der niedriger als der erste vorbestimmte Kontaktdruck ist, der durch das Wandlerelement in einer senkrechten Richtung in Bezug auf die Oberfläche des Säugetierkörpers auf den Säugetierkörper angewendet wird.

4. Behandlungsvorrichtung gemäß einem der vorhergehenden Ansprüche, wobei der dritte vorbestimmte Zustand ein Kontaktdruck ist, der höher als ein zweiter vorbestimmter Kontaktdruck ist, der durch das Wandlerelement in einer senkrechten Richtung in Bezug auf die Oberfläche des Säugetierkörpers auf den Säugetierkörper angewendet wird.

5. Behandlungsvorrichtung gemäß den Ansprüchen 2 bis 4, wobei der erste vorbestimmte Kontaktdruck geringer als der zweite vorbestimmte Kontaktdruck ist.

6. Behandlungsvorrichtung gemäß einem der vorhergehenden Ansprüche, wobei der vorbestimmte Zustand durch die Nachgiebigkeit des nachgiebigen Kopplungselements definiert wird.

7. Behandlungsvorrichtung gemäß einem der vorhergehenden Ansprüche, wobei die Ausgabe ein Ultraschallsignal ist.

8. Behandlungsvorrichtung gemäß einem der vorhergehenden Ansprüche, die ferner eine Datenspeichersteuerung beinhaltet, die angepasst ist, um die Aktivierungsanzahl und -dauer des Wandlerelements für eine Vielzahl von Anwenderprofilen zu bestimmen und aufzuzeichnen.

9. Behandlungsvorrichtung gemäß einem der vorhergehenden Ansprüche, die ferner eine externe Schnittstelle beinhaltet, die angepasst ist, um betriebsfähig mit einer externen Energiequelle und/oder externen Datenspeichereinrichtung verbunden zu werden.

10. Behandlungsvorrichtung gemäß Anspruch 9, wobei die externe Schnittstelle eine Anschlussschnittstelle (Hot-Shoe Interface) ist.

11. Behandlungsvorrichtung gemäß einem der vorhergehenden Ansprüche, die ferner mindestens einen Näherungssensor beinhaltet, der angepasst ist, um die Position der Behandlungsvorrichtung in Bezug auf den Säugetierkörper zu bestimmen, wobei der Näherungssensor angepasst ist, um als Reaktion auf mindestens eine von dem mindestens einen Näherungssensor gemessene vorbestimmte Position selektiv mindestens ein zweites Rückmeldesignal bereitzustellen.

12. Behandlungsvorrichtung gemäß Anspruch 11, wobei das mindestens eine erste und zweite Rückmeldesignal ein beliebiges oder mehrere von einem visuellen und/oder hörbaren und/oder fühlbaren Signal ist.

13. Behandlungsvorrichtung gemäß einem der Ansprüche 11 und 12, das ferner einen ersten Transceiver beinhaltet, der angepasst ist, um das erste und/oder zweite Rückmeldesignal an einen entfernten zweiten Transceiver zu übertragen.

14. Behandlungsvorrichtung gemäß Anspruch 13, wobei der erste Transceiver ferner angepasst ist, um ein Steuersignal zu empfangen, das angepasst ist, um die Ausgabe des Wandlerelements zu steuern.

15. Behandlungsvorrichtung gemäß einem der Ansprüche 11 bis 14, wobei das zweite Rückmeldesignal angepasst ist, um sich als Reaktion auf eine Änderung der Position der Säugetierbehandlungsvorrichtung in Bezug auf den Säugetierkörper im Gebrauch zu ändern.

## Revendications

1. Un appareil de traitement pour une application externe sur un corps de mammifère, comprenant:
un logement,
un élément transducteur, conçu pour fournir une sortie vers et/ou jusque dans le corps de mammifère,
un élément commutateur, conçu pour activer ledit élément transducteur conformément à une première condition prédéterminée et désactiver ledit élément transducteur conformément à une deuxième condition prédéterminée,
un élément de réaction, conçu pour fournir au moins un premier signal de réaction en réponse à au moins une troisième condition prédéterminée, et
un élément de couplage résilient, fait en matériau élastique et conçu pour coupler de façon opérationnelle ledit élément transducteur audit logement et fournir une gamme limitée de trois degrés de liberté entre ledit élément transducteur et ledit logement.

2. Un appareil de traitement selon la revendication 1, dans lequel ladite première condition prédéterminée est une pression de contact égale ou supérieure à une première pression de contact prédéterminée appliquée par ledit élément transducteur sur le corps de mammifère dans un sens perpendiculaire par rapport à la surface du corps de mammifère.

3. Un appareil de traitement selon la revendication 2, dans lequel ladite deuxième condition prédéterminée est une pression de contact inférieure à ladite première pression de contact prédéterminée appliquée par ledit élément transducteur sur le corps de mammifère dans un sens perpendiculaire par rapport à la surface du corps de mammifère.

4. Un appareil de traitement selon une quelconque des revendications précédentes, dans lequel ladite troisième condition prédéterminée est une pression de contact supérieure à une deuxième pression de contact prédéterminée appliquée par ledit élément transducteur sur le corps de mammifère dans un sens perpendiculaire par rapport à la surface du corps de mammifère.

5. Un appareil de traitement selon les revendications 2 à 4, dans lequel ladite première pression de contact prédéterminée est plus petite que ladite deuxième pression de contact prédéterminée.

6. Un appareil de traitement selon une quelconque des revendications précédentes, dans lequel ladite condition prédéterminée est définie par la résilience dudit élément de couplage résilient.

7. Un appareil de traitement selon une quelconque des revendications précédentes, dans lequel ladite sortie est un signal à ultrasons.

8. Un appareil de traitement selon une quelconque des revendications précédentes, comprenant en sus un appareil de commande de stockage de données conçu pour déterminer et journaliser le nombre et la durée d'activation dudit élément transducteur pour une pluralité de profils d'utilisateurs.

9. Un appareil de traitement selon une quelconque des revendications précédentes, comprenant en sus une interface externe conçue pour se connecter de façon opérationnelle à une source d'alimentation externe et/ou à un dispositif de stockage de données externe.

10. Un appareil de traitement selon la revendication 9, dans lequel ladite interface externe est une interface à griffe.

11. Un appareil de traitement selon une quelconque des revendications précédentes, comprenant en sus au moins un capteur de proximité conçu pour déterminer l'emplacement dudit appareil de traitement par rapport au corps de mammifère, dans lequel ledit capteur de proximité est conçu pour fournir de façon sélective au moins un deuxième signal de réaction en réponse à au moins une position prédéterminée qui est mesurée par ledit au moins un capteur de proximité.

12. Un appareil de traitement selon la revendication 11, dans lequel ledit au moins un premier et deuxième signal de réaction est un quelconque ou plusieurs signaux parmi un signal optique et/ou un signal sonore et/ou un signal tactile.

13. Un appareil de traitement selon une quelconque des revendications 11 et 12, comprenant en sus un premier émetteur-récepteur conçu pour transmettre ledit premier et/ou deuxième signal de réaction à un deuxième émetteur-récepteur à distance.

14. Un appareil de traitement selon la revendication 13, dans lequel ledit premier émetteur-récepteur est conçu en sus pour recevoir un signal de commande conçu pour commander la sortie dudit élément transducteur.

15. Un appareil de traitement selon une quelconque des revendications 11 à 14, dans lequel ledit deuxième signal de réaction est conçu pour changer en réponse à un changement d'emplacement de l'appareil de traitement pour mammifère par rapport au corps de mammifère durant l'utilisation.
